# EUROPEAN PATENT APPLICATION

(11) **EP 1 864 661 A1**
(43) Date of publication of application: **12.12.2007**
(21) Application number: 06729992.5
(22) Date of filing: 17.03.2006
(51) Int. Cl.: A61K 31/4172, A61K 38/00, A61P 1/04, A61P 1/10, A61P 43/00

(54) **PROPHYLACTIC/THERAPEUTIC AGENT FOR STRESS-INDUCED BOWEL DISEASE**

(30) Priority: 18.03.2005 JP 2005080053
(71) Applicant: Ajinomoto Co., Inc., Tokyo 104-8315 (JP)
(72) Inventor: TAKAHARA, Yoshiyuki, -ku, Kawasaki-shi, Kanagawa, 2108681 (JP)
(74) Representative: Nicholls, Kathryn Margaret
(86) International application number: PCT/JP2006/306044
(87) International publication number: WO 2006/098524

(57) **Abstract**

Provided are a prophylactic/therapeutic agent for a stress-induced bowel disease, containing, as an active ingredient, at least one kind selected from the group consisting of histidine, a peptide comprising histidine, a precursor thereof and a pharmacologically acceptable salt thereof, a food/beverage having prophylactic/ameliorating action on the disease, a suppressant of the release of a chemical transmitter during stress induction, and a food/beverage having suppressive action on the release of the transmitter.

## Description

### Technical Field

The present invention relates to a prophylactic/therapeutic agent for a stress-induced bowel disease, more specifically to a prophylactic/therapeutic agent for a stress-induced bowel disease, comprising histidine as an active ingredient, a suppressant of the release of a chemical transmitter during stress induction or a prophylactic/therapeutic method for the disease using them. The present invention also relates to a food/beverage for the prevention/amelioration of a stress-induced bowel disease, comprising histidine.

### Background Art

Of bowel diseases, diarrhea develops with various factors, and diarrhea that develops due to severe stress or long-term stress is also known. It has been explained that the central nerve perceives stress and produces excitation of the sympathetic nerve, which in turn causes excitation of the parasympathetic nerve in antagonism against the sympathetic nerve and results in parasympathetic predominant gut motor activation and diarrhea. Not only gut motor acceleration, but also suppression of water absorption from the gut or increase of water secretion from the gut leads to diarrhea. Bowel diseases associated with diarrhea are classified as shown in Table 1, including inflammatory bowel disease (IBD) and others of unknown causes. Stress is considered to be associated with various bowel diseases, and is at least viewed as a disease aggravation factor. Particularly, irritable bowel syndrome (IBS) has been reported to be aggravated by stress (Minerva Med. 2004 Oct; 95(5):443-50, Psychological influences on the irritable bowel syndrome; Gut. 2004 Aug;53(8):1102-8. Altered visceral perceptual and neuroendocrine response in patients with irritable bowel syndrome during mental stress; Curr Psychiatry Rep. 2004 Jun; 6(3):210-5; The interface of psychiatry and irritable bowel syndrome). In these circumstances, there is a demand for the development of a drug that is highly effective and highly safe as a therapeutic or prophylactic agent for various bowel diseases, particularly diarrhea, that develop due to stress.

**(Table 1)**

| Bowel diseases accompanied by diarrhea | | | | | |
|---|---|---|---|---|---|
| Infectious enteritis | | | | | |
| Disease-associated enteritis | IBD | | Inflammatory bowel disease (IBD) | | |
| | Ulcerative colitis | | Ulcerative colitis (UC) | Abdominal pain, mucous and bloody stool | Cause unknown |
| | Crohn's disease | | Crohn's Disease (CD) | Non-continuous lesion, diarrhea | Cause unknown |
| | | | Indeterminate colitis | | |
| | Collagenous enteritis | | Collagenous colitis | Chronic watery diarrhea, subepithelial collagen fiber hyperplasia | |
| | Simple ulcer | | Simple ulcer | Abdominal pain | Cause unknown |
| Irritable bowel syndrome | | | Imitable bowel syndrome (IBS) | Abdominal pain, bowel movement abnormality | Stress is an aggravation factor |
| Functional diarrhea | | | Functional diarrhea | Diarrhea | |
| | | | Antibiotics-associated colitis | | |
| Antibiotic-associated colitis | | Pseudo-membranous colitis | Pseudo-membranous colitis | | |
| | | Hemorrhagic colitis | Hemorrhagic colitis | | |
| Food allergy/eosinophilic gastroenteritis | | | | Abdominal pain, diarrhea | |
| Lactase intolerance | | | Lactase deficiency | Abdominal pain, diarrhea | Small intestine mucosal brush border lactase deficiency |

Meanwhile, reports are available on the feasibility of bowel disease treatment with histidine.
(1) Mitigates ulcerative damage of the intestine by infectious microorganisms and drugs. This is accounted for as an antiinflammatory action derived from the peroxide elimination potential of histidine.
(2) The suppressive action of histidine on the gut water secretion by cholera toxin, a bacterial toxin that causes the diarrhea in cholera, has been reported. This is accounted for by the fact that histidine has suppressive action on the gut water secretion due to increase of cAMP in enterocytes, a mechanism for the development of diarrhea.

However, both only show the possibility of suppression of the effects of adventitious materials or microorganisms on the gut, with no description of the effect of histidine on the diarrhea caused due to the involvement of the central to peripheral nervous systems known as stress (description for US Patent No. 5741807).

### Disclosure of the Invention

Judging from its structure, histidine is thought to bind to peroxides and the inflammatory substance prostaglandin to inhibit the functions thereof, but it seems to have various other physiological actions. It is an object of the present invention to find a new physiological action of histidine to suppress the signals from the stress-perceiving central nervous system to the peripheral nervous system, and to provide a prophylactic/therapeutic agent for a stress-induced bowel disease and a suppressant of the release of a chemical transmitter during stress induction. It is another object of the present invention to provide a food/beverage for prevention/amelioration of the bowel disease and for the suppression of the release.

The present inventors diligently investigated to solve the above-described problems and, as a result, found that histidine prevented and effectively ameliorated a stress-induced bowel disease, and developed the present invention.

Accordingly, the present invention relates to:
(1) A prophylactic/therapeutic agent for a stress-induced bowel disease, comprising, as an active ingredient, at least one kind selected from the group consisting of histidine, a peptide comprising histidine, a precursor thereof and a pharmacologically acceptable salt thereof.
(2) The prophylactic/therapeutic agent of (1), wherein the stress-induced bowel disease is stress-induced diarrhea.
(3) The prophylactic/therapeutic agent of (1), wherein the stress-induced bowel disease is at least one kind selected from the group consisting of ulcerative colitis, Crohn's disease, irritable bowel syndrome, functional diarrhea and stress-induced exacerbation of these bowel diseases.
(4) The prophylactic/therapeutic agent of (1), wherein the stress-induced bowel disease is a bowel disease resulting from the release of a chemical transmitter during stress induction.
(5) The prophylactic/therapeutic agent of (4), wherein the bowel disease resulting from the release of a chemical transmitter during stress induction is stress-induced diarrhea.
(6) The prophylactic/therapeutic agent of (4) or (5), wherein the chemical transmitter is at least one kind selected from among catecholamine, serotonin and histamine.
(7) A suppressant of the release of a chemical transmitter during stress induction, comprising, as an active ingredient, at least one kind selected from the group consisting of histidine, a peptide comprising histidine, a precursor thereof and a pharmacologically acceptable salt thereof.
(8) The release suppressant of (7), wherein the chemical transmitter is at least one kind selected from among catecholamine, serotonin and histamine.
(9) A food/beverage for prevention/amelioration of a stress-induced bowel disease, comprising, as an active ingredient, at least one kind selected from the group consisting of histidine, a peptide comprising histidine, a precursor thereof and a pharmacologically acceptable salt thereof.
(10) A food/beverage comprising at least one kind selected from the group consisting of histidine, a peptide comprising histidine, a precursor thereof and a pharmacologically acceptable salt thereof, having prophylactic/ameliorating action on a stress-induced bowel disease, and bearing a label stating that it is intended to prevent/ameliorate a stress-induced bowel disease.
(11) The food/beverage of (9) or (10), wherein the stress-induced bowel disease is stress-induced diarrhea.
(12) The food/beverage of (9) or (10), wherein the stress-induced bowel disease is at least one kind selected from the group consisting of ulcerative colitis, Crohn's disease, irritable bowel syndrome, functional diarrhea and stress-induced exacerbation of these bowel diseases.
(13) The food/beverage of (9) or (10), wherein the stress-induced bowel disease is a bowel disease resulting from the release of a chemical transmitter during stress induction.
(14) The food/beverage of (13), wherein the bowel disease resulting from the release of a chemical transmitter during stress induction is stress-induced diarrhea.
(15) The food/beverage of (13) or (14), wherein the chemical transmitter is at least one kind selected from among catecholamine, serotonin and histamine.
(16) The food/beverage of any of (9) to (15), which is a food with health claims or a dietary supplement.
(17) The food/beverage of (16), wherein the above-mentioned food with health claims is a food for specified health uses or a food with nutrient function claims.
(18) A food/beverage comprising at least one kind selected from the group consisting of histidine, a peptide comprising histidine, a precursor thereof and a pharmacologically acceptable salt thereof, having suppressive action on the release of a chemical transmitter during stress induction, and bearing a label stating that it is intended to suppress the release of a chemical transmitter during stress induction.
(19) A commercial package comprising a composition comprising, as an active ingredient, at least one kind selected from the group consisting of histidine, a peptide comprising histidine, a precursor thereof and a pharmacologically acceptable salt thereof, and a printed matter stating that the composition can be used, or should be used, to prevent/treat a stress-induced bowel disease.
(20) A commercial package comprising a composition comprising, as an active ingredient, at least one kind selected from the group consisting of histidine, a peptide comprising histidine, a precursor thereof and a pharmacologically acceptable salt thereof, and a printed matter stating that the composition can be used, or should be used, to suppress the release of a chemical transmitter during stress induction.
(21) A commercial package comprising a food/beverage comprising at least one kind selected from the group consisting of histidine, a peptide comprising histidine, a precursor thereof and a pharmacologically acceptable salt thereof, and a printed matter showing matters concerning its use for prevention/amelioration of a stress-induced bowel disease.
(22) A commercial package comprising a food/beverage comprising at least one kind selected from the group consisting of histidine, a peptide comprising histidine, a precursor thereof and a pharmacologically acceptable salt thereof, and a printed matter showing matters concerning its use for suppression of the release of a chemical transmitter during stress induction.
(23) A prophylactic or therapeutic method for a stress-induced bowel disease, comprising administering an effective amount of at least one kind selected from the group consisting of histidine, a peptide comprising histidine, a precursor thereof and a pharmacologically acceptable salt thereof to a subject of administration.
(24) A method of suppressing the release of a chemical transmitter during stress induction, comprising administering an effective amount of at least one kind selected from the group consisting of histidine, a peptide comprising histidine, a precursor thereof and a pharmacologically acceptable salt thereof to a subject of administration.
(25) A prophylactic or ameliorating method for a stress-induced bowel disease, comprising taking a food/beverage containing an effective amount of at least one kind selected from the group consisting of histidine, a peptide comprising histidine, a precursor thereof and a pharmacologically acceptable salt thereof to a subject of administration.
(26) A method of suppressing the release of a chemical transmitter during stress induction, comprising taking a food/beverage comprising an effective amount of at least one kind selected from the group consisting of histidine, a peptide comprising histidine, a precursor thereof and a pharmacologically acceptable salt thereof to a subject of administration.
(27) A use of histidine, a peptide comprising histidine, a precursor thereof or a pharmacologically acceptable salt thereof for producing a prophylactic/therapeutic agent for a stress-induced bowel disease.
(28) A use of histidine, a peptide comprising histidine, a precursor thereof or a pharmacologically acceptable salt thereof for producing a suppressant of the release of a chemical transmitter during stress induction.
(29) A use of histidine, a peptide comprising histidine, a precursor thereof or a pharmacologically acceptable salt thereof for producing a food/beverage having prophylactic/ameliorating action on a stress-induced bowel disease, and bearing a label stating that it is intended to prevent/ameliorate a stress-induced bowel disease.
(30) A use of histidine, a peptide comprising histidine, a precursor thereof or a pharmacologically acceptable salt thereof for producing a food/beverage having suppressive action on the release of a chemical transmitter during stress induction, and bearing a label stating that it is intended to suppress the release of a chemical transmitter during stress induction.

### Best Mode for Embodying the Invention

Described below are embodiments of the present invention.

Although the histidine used in the present invention may be any of the L-form, the D- form, and a mixture thereof (for example, racemate), the L- form is preferably used.

Although the histidine used may be any histidine, peptide comprising histidine, or precursor thereof, obtained by peptide synthesis or synthesis using Escherichia coli, these are not to be construed as limiting.

The peptide comprising histidine in the present invention means a peptide comprising one or more amino acids having an imidazole skeleton, capable of becoming histidine or a histidine precursor. The peptide is normally a peptide having not more than 20 amino acids; particularly, the amino acid number is preferably not more than 10, more preferably not more than 3, still more preferably 2.

A precursor of histidine or a peptide comprising a histidine refers to a precursor that converts to histidine or a peptide comprising histidine after being metabolized in the body; carnosine, which consists of L-histidine and β alanine, anserine, which consists of β alanine and 1-methylhistidine, valenin, which consists of β alanine and 3-methylhistidine, homocarnosine (γ aminobutyryl-L-histidine), N-acetyl-L-histidine, N-acetylcarnosine, N-acetylanserine and the like can be mentioned.

The histidine, peptide comprising histidine and precursor thereof, used in the present invention, can be used not only as a free form, but also in the form of a salt. As the salt, a salt with an inorganic base, a salt with an inorganic acid, a salt with an organic acid and the like can be mentioned; it is preferable to choose a salt acceptable as a pharmaceutical or a food/beverage. As the salt with an inorganic base, salts with alkali metals such as sodium, potassium and lithium, salts with alkaline earth metals such as calcium and magnesium, ammonium salts and the like can be mentioned. As the salt with an inorganic acid, salts with hydrohalogenic acids (hydrochloric acid, hydrobromic acid, hydroiodic acid and the like), sulfuric acid, nitric acid, phosphoric acid and the like can be mentioned. As the salt with an organic acid, salts with formic acid, acetic acid, propionic acid, oxalic acid, succinic acid, maleic acid, fumaric acid, and citric acid can be mentioned.

With respect to a prophylactic/therapeutic agent for a stress-induced bowel disease, comprising, as an active ingredient, at least one kind selected from the group consisting of histidine, a peptide comprising histidine, a precursor thereof and a pharmacologically acceptable salt thereof, treatment refers to an amelioration of the disease, wherein amelioration is a concept including the prevention of progression (aggravation) of the disease.

In the present invention, "a stress-induced bowel disease" is not subject to limitation, as long as it is a disease of the intestine induced by stress. Specifically, the stress-induced bowel disease is a disease of parts of the intestine such as the small intestine (duodenum, jejunum, ileum) and the large intestine (colon, rectum), preferably a disease of the large intestine. As the bowel disease in the present invention, stress-induced diarrhea, ulcerative colitis, Crohn's disease, irritable bowel syndrome, functional diarrhea and stress-induced exacerbation of these bowel diseases can be mentioned. A bowel disease also develops due to the release of a chemical transmitter during stress induction.

"Stress" refers to a tense state in the body in response to an inductive harmful factor in the body (stressor). "Stress-induced diarrhea" means diarrhea caused by physically or mentally exerted stress, rather than infectious diarrhea, which is caused by an infectious microorganism, drug and cytotoxin and the like; for example, by this is meant diarrhea characterized by accentuation of peristalsis and accentuation of secretion of water or electrolytes, caused by a stressor such as noise, cold, trauma, anxiety or tension.

"A bowel disease resulting from the release of a chemical transmitter during stress induction" refers to a bowel disease resulting from the release of a chemical transmitter as described below from the peripheral nerves around the gut or from body defense-related cells such as monocytes under the control of the central nerve or hormones under stress loading conditions.

As the "chemical transmitter" released during stress induction, catecholamines (noradrenaline, adrenaline, dopamine and the like), histamine, serotonin, acetylcholine and leukotrienes, prostaglandins, nitrogen monoxide (NO) and the like can be mentioned. Desirably, catecholamines (noradrenaline, adrenaline, dopamine and the like), serotonin, histamine and the like can be mentioned. Polypeptides such as substance P, VIP, various chemokines and cytokines are also released. The behavior of chemical transmitters during stress induction has been reported (J Physiol. 2003 Dec 15;553(Pt 3):959-66. Epub 2003 Oct 10. Acute stress modulates the histamine content of mast cells in the gastrointestinal tract through interleukin-1 and corticotropin-releasing factor release in rats; Gut. 2004 Aug;53(8):1102-8 Altered visceral perceptual and neuroendocrine response in patients with irritable bowel syndrome during mental stress).

The present invention provides a suppressant of the release of the above-described chemical transmitters during stress induction. The present invention also provides a method of suppressing the release of a chemical transmitter during stress induction, comprising administering an effective amount of at least one kind selected from the group consisting of histidine, a peptide comprising histidine, a precursor thereof and a pharmacologically acceptable salt thereof to a subject of administration, and a use of histidine, a peptide comprising histidine, a precursor thereof or a pharmacologically acceptable salt thereof for producing a suppressant of the release of a chemical transmitter during stress induction.

The present invention provides a suppressant of the proteolysis of the mucosa of the large intestine during stress induction. Suppression of proteolysis means that the promotion of proteolysis during stress induction is suppressed.

In the present invention, histidine, a peptide comprising histidine, a precursor thereof and a pharmacologically acceptable salt thereof has an excellent prophylactic/therapeutic effect on a stress-induced bowel disease in mammals (for example, mice, rats, hamsters, rabbits, cats, dogs, cattle, sheep, monkeys, humans and the like), and also has a suppressive effect on the release of a chemical transmitter during stress induction, and is therefore useful as a prophylactic/therapeutic agent and prophylactic/ameliorating food/beverage for a stress-induced bowel disease in various animals, and as a suppressant of the release of a chemical transmitter and a food/beverage having the suppressive action on the release.

Although the method of administration (or method of ingestion as a food) of the prophylactic/therapeutic agent or release suppressant of the present invention is not subject to limitation, and may be oral administration or a parenteral administration (ingestion) such as drip infusion administration or injection administration (transvenous administration), oral administration is preferable because the active ingredient thereof is an amino acid. As the preparation for oral administration, granules, fine granules, powders, coated tablets, tablets, suppositories, triturates, (micro)capsules, chewable tablets, syrups, juices, solutions, suspensions, emulsions and the like can be adopted; as the injectable preparation, a dosage form for common use for pharmaceutical preparations, such as for direct venous injection, for drip infusion administration, or a preparation for extending the release of an active substance, can be adopted.

In the prophylactic/therapeutic agent or release suppressant of the present invention, in the case of oral administration, the dosage varies depending on the recipient patient's symptoms, age, sex, method of administration and the like, and is normally from about 0.1 g/day to 5 g/day, preferably about 0.5 g/time to 2 g/time given twice a day, more preferably about 1.5 g/time given twice a day, per day for an adult based on histidine.

Histidine refers to the weight of the histidine per se when the histidine itself is contained, and, when it contains a peptide comprising histidine and a precursor thereof, means histidine converted from all substances capable of becoming histidine by hydrolysis and the like thereof.

The above-described daily dose can be administered at one time or in several divided portions. It does not matter when it is administered before meals, after meals, or between meals. Duration of administration is not subject to limitation.

Regarding the dosage in the case of a parenteral ingestion such as drip infusion administration or injection administration (transvenous administration), a dosage about one tenth to one second of the preferred range of dosage for the foregoing oral administration can be administered.

The prophylactic/therapeutic agent or release suppressant of the present invention can be prepared by making a pharmaceutical preparation by a conventional method. As required from the viewpoint of pharmaceutical making, various pharmacologically acceptable substances for pharmaceutical making (as auxiliaries and the like) can be formulated. A substance for pharmaceutical making can be chosen as appropriate according to the dosage form of the preparation; for example, excipients, diluents, additives, disintegrants, binders, coating agents, lubricants, sliding agents, glazing agents, flavoring agents, sweetening agents, solubilizers and the like can be mentioned. Furthermore, as specific examples of the substance for pharmaceutical making, magnesium carbonate, titanium dioxide, lactose, mannitol and other saccharides, talc, cow's milk protein, gelatin, starch, cellulose and derivatives thereof, animal or vegetable oils, polyethylene glycol, and solvents, for example, sterile water and monohydric or polyhydric alcohols, for example, glycerol, and the like can be mentioned.

The content of histidine in the prophylactic/therapeutic agent or release suppressant of the present invention is normally 1 to 20% by weight, preferably 1 to 10% by weight, more preferably 2 to 5% by weight.

The prophylactic/therapeutic agent or release suppressant of the present invention can also be prepared as a pharmaceutical formulation not only by a conventional method, but also by various forms of pharmaceutical preparations that will be developed in the future. For the preparations, methods that will be developed in the future can be adopted as appropriate.

In the case of the food/beverage of the present invention for prevention/amelioration of a stress-induced bowel disease, or for suppression of the release of a chemical transmitter, the form thereof is not subject to limitation, as long as an amount of histidine, a peptide comprising histidine, a precursor thereof and a pharmacologically acceptable salt thereof necessary to exhibit the preventive/ameliorating effect on a stress-induced bowel disease or suppressive effect on the release of a chemical transmitter is contained in the food/beverage.

The ingestion form of the food/beverage of the present invention is not subject to limitation, and may be oral ingestion or parenteral ingestion, but oral ingestion is preferable because the active ingredient thereof is an amino acid. In the food/beverage of the present invention, in the case of oral ingestion, the amount ingested varies depending on the recipient's symptoms, age, sex, method of ingestion and the like, is normally from about 0.1 g/day to 10 g/day, preferably from about 1 g/day to 5 g/day, more preferably from about 2 g/day to 4 g/day, per day for an adult based on histidine.

The above-described daily dose can be ingested at one time or in several divided portions. Duration of ingestion is not subject to limitation.

Regarding the amount ingested in the case of parenteral ingestion, an amount about one twentieth to one fifth of the foregoing preferred range of amount ingested for oral ingestion can be administered.

Because the active ingredient thereof is an amino acid, the present invention has excellent safety, and can be conveniently used even in the form of a food/beverage. The food/beverage includes health foods, food additives, nutrition supplementary foods and the like ingested for the purpose of supplementing nutrition. In using the present invention in a food/beverage, there is no particular difficulty; for example, the invention can be taken in a mixture in juices, cow's milk, confectionery, jellies and the like. When the invention is used as a health food, food additive, nutrition supplementary food and the like, it can be prepared as forms, for example, tablets, capsules, triturates, granules, suspensions, chewable tablets, syrups and the like.

The content of histidine in the food/beverage of the present invention is normally 1 to 20% by weight, preferably 1 to 10% by weight, more preferably 2 to 5% by weight.

Such a food can also be provided as a food with health claims or a dietary supplement. This food with health claims includes a food/beverage bearing a label stating that it is intended to prevent/ameliorate a stress-induced bowel disease, particularly a food for specified health uses, or a food with nutrient function claims and the like.

Furthermore, a commercial package comprising a composition comprising, as an active ingredient, at least one kind selected from the group consisting of histidine, a peptide comprising histidine, a precursor thereof and a pharmacologically acceptable salt thereof, and a printed matter stating that the composition can be used, or should be used, to prevent/treat a stress-induced bowel disease, is also included in the present invention.

A prophylactic or therapeutic method for a stress-induced bowel disease, comprising administering an effective amount of at least one kind selected from the group consisting of histidine, a peptide comprising histidine, a precursor thereof and a pharmacologically acceptable salt thereof to a subject of administration, and a use of histidine, a peptide comprising histidine, a precursor thereof or a pharmacologically acceptable salt thereof for producing a prophylactic/therapeutic agent for a stress-induced bowel disease are also included in the present invention.

### Examples

The present invention is hereinafter described in more detail by means of the following Examples. The following Examples are for illustrative purposes only and never limit the scope of the invention.

### (Example 1)

After being preliminarily reared on an ordinary diet (CRF-1, Charles River Japan Inc.) for 1 week, Wistar rats (4 weeks of age, male) were fed an ordinary diet (control group) or a histidine diet prepared by mixing finely milled L-histidine hydrochloride at 2% by weight in the ordinary diet (histidine dosing group) for 2 weeks. In the mornings of day 15, day 16, and day 17 after the start of the rearing with the histidine diet, stress loading was performed by the method described below, and the feces excreted by the rats were examined for weight and description.

### <Stress loading>

After being allowed to stand in a small individual cage (13.5x27x12 cm) for about 2 hours, each rat was transferred to a constraint (stress) cage having a size such that the rat cannot move (5x17x4 cm), and further allowed to stand for 2 hours, whereby stress loading was performed (control stress group, histidine dosing stress group). The feces excreted by the rat were examined for description, number, and weight. The stress loading and fecal examination were performed on each individual for three consecutive days.

The results are shown in Table 2 and Table 3. Eight rats were used for each group. The histidine dosing group, compared to the control group, exhibited a reduction in total fecal weight, a reduction in the number of individuals with diarrhea, a reduction in the number of individuals with soft feces and a reduction in soft fece defecation rate (number of normal feces: number of soft feces). It is known that as stress is continuously loaded, the stress attenuates gradually; this study showed the same tendency, and the difference between the histidine dosing group and the control group tended to decrease. From the results shown above, a suppressive effect of histidine administration on diarrhea was confirmed.

**(Table 2)**

| Effects of histidine diet on stress loading using a stress cage | | | | | | | | | | | | | |
|---|---|---|---|---|---|---|---|---|---|---|---|---|---|
| | | Number of feces | | | | | | | | | Fecal weight (g) | | |
| | | Day 1 | Day 1 | Day 1 | Day 2 | Day 2 | Day 2 | Day 3 | Day 3 | Day 3 | Total weight | Total weight | Total weight |
| Histidine dosing stress group | ID NO. | normal feces | soft feces | diarrhea | normal feces | soft feces | diarrhea | normal feces | soft feces | diarrhea | Day 1 | Day 2 | Day 3 |
| | 1 | 3 | 3 | 0 | 6 | 0 | 0 | 6 | 0 | 0 | 1.77 | 1.28 | 1.64 |
| | 2 | 0 | 0 | 0 | 6 | 1 | 0 | 3 | 0 | 0 | 0.00 | 1.26 | 0.41 |
| | 9 | 6 | 0 | 0 | 4 | 0 | 0 | 6 | 0 | 0 | 1.01 | 0.58 | 1.15 |
| | 12 | 3 | 1 | 0 | 2 | 0 | 0 | 1 | 1 | 0 | 1.65 | 0.56 | 0.46 |
| | 15 | 1 | 3 | 1 | 10 | 3 | 2 | 6 | 4 | 1 | 1.94 | 4.02 | 3.27 |
| | 33 | 11 | 0 | 0 | 8 | 2 | 0 | 4 | 1 | 0 | 2.23 | 2.38 | 1.00 |
| | 5 | 10 | 1 | 0 | 3 | 0 | 0 | 6 | 4 | 0 | 2.82 | 0.25 | 2.22 |
| | 13 | 5 | 3 | 0 | 5 | 5 | 0 | 6 | 5 | 0 | 2.46 | 3.48 | 3.99 |
| Control (ordinary diet) stress group | ID NO. | normal feces | soft feces | diarrhea | normal feces | soft feces | diarrhea | normal feces | soft feces | diarrhea | | | |
| | 18 | 4 | 4 | 4 | 3 | 8 | 1 | 3 | 8 | 0 | 3.71 | 3.27 | 2.67 |
| | 19 | 3 | 2 | 3 | 3 | 3 | 0 | 7 | 4 | 1 | 2.41 | 1.66 | 3.17 |
| | 22 | 0 | 4 | 0 | 5 | 3 | 0 | 3 | 2 | 2 | 1.72 | 2.61 | 2.57 |
| | 23 | 2 | 2 | 0 | 4 | 3 | 0 | 5 | 1 | 0 | 1.99 | 1.51 | 1.92 |
| | 25 | 1 | 10 | 1 | 1 | 4 | 0 | 4 | 4 | 0 | 3.11 | 1.38 | 1.83 |
| | 29 | 2 | 3 | 0 | 2 | 3 | 0 | 2 | 2 | 0 | 1.69 | 1.42 | 1.36 |
| | 32 | 3 | 3 | 2 | 3 | 4 | 2 | 3 | 5 | 0 | 2.56 | 3.37 | 2.94 |
| | 28 | 2 | 10 | 1 | 4 | 3 | 4 | 4 | 2 | 2 | 3.26 | 3.68 | 2.67 |

**(Table 3)**

| Suppressive effects of histidine diet on diarrhea under stress loading using a stress cage | | | | |
|---|---|---|---|---|
| Stress loading | | Non-stress group | Control stress group | Histidine dosing stress group |
| Day 1 | Number of individuals examined | 8 | 8 | 8 |
| | Number of individuals with diarrhea | 0 | 5 | 1 |
| | Number of individuals with soft feces | 0 | 8 | 5 |
| | Number of individuals without diarrhea or soft feces | 8 | 0 | 3 |
| | Number of normal feces: number of soft feces | | 1:2.24 | 1:0.28 |
| Day 2 | Number of individuals examined | 8 | 8 | 8 |
| | Number of individuals with diarrhea | 0 | 3 | 1 |
| | Number of individuals with soft feces | 0 | 8 | 4 |
| | Number of individuals without diarrhea or soft feces | 8 | 0 | 4 |
| | Number of normal feces: number of soft feces | | 1:1.24 | 1:0.25 |
| Day 3 | Number of individuals examined | 8 | 8 | 8 |
| | Number of individuals with diarrhea | 0 | 3 | 1 |
| | Number of individuals with soft feces | 0 | 8 | 5 |
| | Number of individuals without diarrhea or soft feces | 8 | 0 | 3 |
| | Number of normal feces: number of soft feces | | 1:0.90 | 1:0.40 |

| | | | | |
|---|---|---|---|---|
| Note: Individuals with diarrhea overlap individuals with soft feces. | | | | |

The large intestines of stress-loaded rats were collected and frozen in liquid nitrogen on day 17, and stored at -80°C until use. Extracted from the frozen samples by the following protocol were noradrenaline, histamine, serotonin and 3-methylhistidine, which were then quantified by LC/MS.

Each frozen rat tissue (large intestine) stored at -80°C was disrupted using CryoPress (manufactured by Microtec Co., Ltd.), and 1 mL of methanol per 100 mg at -80°C was added. The mixture was disrupted using FastPrep (30 sec x 2 times), and 0.8 mL of the disruption liquid was recovered. 1 mL of cold chloroform was added thereto, and the mixture was stirred using Vortex for 1 minute, after which 1 mL of cold H₂O was added. After the mixture was further stirred using Vortex for 1 minute, it was allowed to stand for 30 minutes. After being allowed to stand, the mixture was centrifuged at 1000 rpm for 3 minutes at 4°C. 1 mL of the about 1.5 mL H₂O/MeOH layer was recovered.
This was evaporated to dryness using a centrifugal evaporator. After being dissolved in 100 µL of MilliQ water, the dry product was converted to an AQC derivative, diluted 2 fold with 0.1% formic acid, and assayed using API4000LC-MS/MS.

The tissue contents of noradrenaline, histamine, serotonin and 3-methylhistidine are shown in Table 4.

The tissue contents of noradrenaline, which is used for signal transduction in the sympathetic nerve, in the large intestine increased due to stress loading, suggesting that histidine may have suppressive action.

Also, in the large intestine, the tissue contents of histamine and serotonin increased due to stress loading, and this increase tended to be suppressed by histidine administration. Histamine and serotonin are released from the peripheral nerves around the gut and body defense-related cells such as monocytes under the control of the central nervous system and hormones. These behaviors of noradrenaline, histamine and serotonin in the large intestine demonstrate that histidine has suppressive action on the signals from the stress-perceiving central nervous system to the peripheral nervous system.

3-methylhistidine likewise increased with stress loading, and this increase was suppressed by histidine administration.
It has been reported that stress (mental) promotes the degradation of the mucosa of the stomach and the large intestine (J Pineal Res. 2004 Sep;37(2):113-21). 3-methylhistidine is contained in protein and released upon the degradation thereof. Because its content in the blood is essentially low with little variation, 3-methylhistidine is used as an index for proteolysis (Kidney Int Suppl. 1983 Dec;16:S2-8, Ciba Found Symp. 1979;(75):307-29). An increase in 3-methylhistidine in the large intestine due to stress loading indicates an abnormality of the large intestine. From these facts, it is evident that protein collapse occurs in the large intestine during stress induction, and that histidine administration suppresses proteolysis in the large intestine.

The behavior of noradrenaline, histamine, serotonin and 3-methylhistidine increasing during stress induction was remarkably observed in the large intestine. Therefore, it can be thought that gut abnormalities due to stress occur mainly in the large intestine, and that the symptomatic manifestation of diarrhea is suppressed by administering histidine.

**(Table 4)**

| Noradrenaline, histamine, serotonin and 3-methylhistidine contents in the large intestine | | |
|---|---|---|
| Noradrenaline | | |
| Group | Sample Name | Tissue wet weight |
| 1 | Stress- His diet- | 1.00 |
| 2 | Stress+ His diet- | 1.14 |
| 3 | stress+ His diet+ | 0.96 |

| Histamine | | |
|---|---|---|
| Group | Sample Name | Tissue wet weight |
| 1 | Stress- His diet- | 5.62 |
| 2 | Stress+ His diet- | 9.51 |
| 3 | Stress+ His diet+ | 6.61 |

| Serotonin | | |
|---|---|---|
| Group | Sample Name | Tissue wet weight |
| 1 | Stress- His diet- | 5.99 |
| 2 | Stress+ His diet- | 7.66 |
| 3 | Stress+ His diet+ | 6.63 |

| 3-methylhistidine | | |
|---|---|---|
| Group | Sample Name | Tissue wet weight |
| 1 | Stress- His diet- | 0.13 |
| 2 | Stress+ His diet- | 0.43 |
| 3 | Stress+ His diet+ | 0.13 |

| | | |
|---|---|---|
| Unit of measurement: nmol/g For each group: N=8 | | |

### (Example 2)

After being preliminarily reared on an ordinary diet (CRF-1, Charles River Japan Inc.) for 1 week, Wistar rats (6 weeks of age, male) received histidine given by gavage at a frequency of twice a day (morning and evening) on day 8 and day 9 after the start of the preliminary rearing. A 5 mL dosing volume of 0.5% methylcellulose solution (control solution) for the control group, or a solution of L-histidine hydrochloride dissolved (partially suspended) in 0.5% methylcellulose solution to a content ratio of 1.5 g per kg body weight of each rat for the histidine dosing group, was orally administered using a sonde. Stress loading was performed using a stress cage in the same manner as Example 1. That is, after each rat was transferred to a small individual cage (13.5x27x12 cm) in the morning of day 10, and allowed to stand for 2 hours, the same histidine solution or control solution as those given on the previous day were once orally administered. After being allowed to stand in a small individual cage for 2 hours, each rat was transferred to a constraint cage (5x17x4 cm) and allowed to stand for 1 hour, whereby stress loading was performed. The feces excreted by the rat were examined for description, number, and weight.

The results are shown in Table 5 and Table 6. Eight rats were used for each group. The histidine dosing group, compared to the control group, exhibited a reduction in total fecal weight and a reduction in the number of individuals with diarrhea. From the results shown above, a suppressive effect of histidine administration on diarrhea was confirmed.

**(Table 5)**

| Effects of histidine bolus oral administration on stress loading using a stress cage | | | | | | |
|---|---|---|---|---|---|---|
| | Identification number | Body weight | Number of normal feces | Number of soft feces | Onset of diarrhea | Total fecal weight |
| Non-stress group | 1-1 | 228.7 | 1 | 0 | none | 0.147 |
| | 1-2 | 238.2 | 0 | 0 | none | 0 |
| | 1-3 | 239.9 | 0 | 0 | none | 0 |
| | 1-4 | 241.7 | 0 | 0 | none | 0 |
| | 1-5 | 242.4 | 0 | 0 | none | 0 |
| | 1-6 | 247.7 | 0 | 0 | none | 0 |
| | 1-7 | 250.4 | 0 | 0 | none | 0 |
| | 1-8 | 255 | 0 | 0 | none | 0 |
| | Mean | 252.8 | 0 | 0 | 0 individual | 0.02 |
| Control stress group | 2-1 | 242.9 | 0 | 7 | diarrhea | 5.233 |
| | 2-2 | 244.4 | 3 | 5 | none | 1.867 |
| | 2-3 | 252.3 | 1 | 3 | diarrhea | 2.818 |
| | 2-4 | 245.3 | 0 | 4 | none | 0.643 |
| | 2-5 | 248.2 | 0 | 4 | diarrhea | 2.178 |
| | 2-6 | 257.8 | 0 | 7 | none | 1.414 |
| | 2-7 | 255.7 | 0 | 8 | none | 2.847 |
| | 2-8 | 252.5 | 0 | 7 | none | 2.015 |
| | Mean | 249.4 | 0.5 | 5.6 | 3 individuals | 2.38 |
| Histidine dosing stress group | 3-1 | 237.4 | 0 | 12 | none | 2.379 |
| | 3-2 | 239.8 | 0 | 8 | none | 2.7 |
| | 3-3 | 239.8 | 0 | 3 | none | 0.852 |
| | 3-4 | 250.8 | 1 | 5 | none | 2.077 |
| | 3-5 | 246.2 | 0 | 6 | none | 1.515 |
| | 3-6 | 249.2 | 0 | 6 | none | 1.975 |
| | 3-7 | 250.3 | 0 | 4 | none | 0.72 |
| | 3-8 | 259.7 | 0 | 4 | none | 0.94 |
| | Mean | 246.7 | 0.125 | 6 | 0 individual | 1.64 |

| | | | | | | |
|---|---|---|---|---|---|---|
| Fecal weight, g | | | | | | |

**(Table 6)**

| Suppression of diarrhea due to stress loading using a stress cage by histidine bolus oral administration | | | |
|---|---|---|---|
| | Non-stress group | Control stress group | Histidine dosing stress group |
| Individuals examined | 8 | 8 | 8 |
| Number of individuals with diarrhea | 0 | 3 | 0 |
| Number of individuals with soft feces | 0 | 8 | 8 |
| Number of individuals without diarrhea or soft feces | 8 | 0 | 0 |

### (Example 3)

After being preliminarily reared on an ordinary diet (CRF-1, Charles River Japan Inc.) for 1 week, Wistar rats (6 weeks of age, male) received histidine given by gavage at a frequency of twice a day (morning and evening) on day 8 and day 9 as described above. A 5 mL dosing volume of 0.5% methylcellulose solution (control solution) for the control group, or a solution of L-histidine hydrochloride dissolved (partially suspended) in 0.5% methylcellulose solution to a content ratio of 1.5 g per kg body weight of each rat for the histidine dosing group, was orally administered using a sonde. In the morning of day 10, the same histidine solution or control solution as those given on the previous day was once orally administered. One hour later, under low-dose ether anesthesia, stress loading was performed by constraining the front legs with a tape of the Scotch brand. After each animal was allowed to stand for 1 hour in an individual cage, the feces excreted by the rat were examined for description, number, and weight.

The results are shown in Table 7 and Table 8. Eight rats were used for each group. The histidine dosing group, compared to the control group, exhibited a reduction in total fecal weight and a reduction in soft feces defecation rate (ratio of number of soft feces to number of normal feces). From the results shown above, a suppressive effect of histidine administration on diarrhea was confirmed.

**(Table 7)**

| Effects of histidine administration on stress loading by front leg constraint | | | | | | |
|---|---|---|---|---|---|---|
| | Identification number | Body weight | Number of normal feces | Number of soft feces | Onset of diarrhea | Total fecal weight |
| Non-stress group | 1-1 | 233.2 | 0 | 0 | none | 0 |
| | 1-2 | 244.7 | 1 | 0 | none | 0.55 |
| | 1-3 | 228.1 | 2 | 0 | none | 0.43 |
| | 1-4 | 253.4 | 0 | 0 | none | 0 |
| | 1-5 | 256.3 | 0 | 0 | none | 0 |
| | 1-6 | 248.2 | 0 | 0 | none | 0 |
| | 1-7 | 256.1 | 0 | 0 | none | 0 |
| | 1-8 | 263.7 | 0 | 0 | none | 0 |
| | Mean | 256 | 0.38 | 0 | 0 | 0.122 |
| Control stress group | 2-1 | 242.4 | | | | dead |
| | 2-2 | 249.8 | 2 | 5 | none | 2.64 |
| | 2-3 | 246.2 | 0 | 3 | none | 0.79 |
| | 2-4 | 253 | 0 | 4 | none | 2.85 |
| | 2-5 | 253.4 | 0 | 6 | none | 1.65 |
| | 2-6 | 258.4 | 0 | 6 | none | 3.72 |
| | 2-7 | 255.7 | 0 | 3 | none | 1.45 |
| | 2-8 | 262.7 | 0 | 6 | none | 2.28 |
| | Mean | 260.2 | 0.29 | 4.71 | 0 | 2.2 |
| Histidine dosing stress group | 3-1 | 246.7 | 4 | 5 | none | 2.12 |
| | 3-2 | 242.1 | 2 | 2 | none | 0.73 |
| | 3-3 | 246.8 | 0 | 2 | none | 1.03 |
| | 3-4 | 250 | 0 | 0 | none | 0 |
| | 3-5 | 252.7 | 0 | 6 | none | 2.18 |
| | 3-6 | 253.9 | 0 | 6 | none | 2.47 |
| | 3-7 | 257.9 | 1 | 4 | none | 2.63 |
| | 3-8 | 261.5 | 0 | 5 | none | 2.31 |
| | Mean | 257.8 | 0.88 | 3.75 | 0 | 1.68 |

| | | | | | | |
|---|---|---|---|---|---|---|
| Fecal weight, g | | | | | | |

**(Table 8)**

| Suppressive effects of histidine on onset of soft feces due to stress loading by front leg constraint | | | |
|---|---|---|---|
| | Mean number of normal feces | Mean number of soft feces | Number of normal feces:number of soft feces |
| Control stress group | 0.29 | 4.71 | 1:16.2 |
| Histidine dosing stress group | 0.88 | 3.75 | 1:4.2 |

### Industrial Applicability

According to the present invention, a prophylactic/therapeutic agent for a stress-induced bowel disease, particularly stress-induced diarrhea, ulcerative colitis, Crohn's disease, irritable bowel syndrome, functional diarrhea and stress-induced exacerbation of these bowel diseases, can be provided. The prophylactic/therapeutic agent of the present invention has excellent safety and can be administered for a long time, and because the active ingredient thereof is an amino acid, it is highly safe and produces almost no adverse reactions, and can therefore also be used as a food/beverage.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2005-080053 filed in Japan, the contents of which are incorporated in full herein by this reference.

## Claims

1. A prophylactic/therapeutic agent for a stress-induced bowel disease, comprising, as an active ingredient, at least one kind selected from the group consisting of histidine, a peptide comprising histidine, a precursor thereof and a pharmacologically acceptable salt thereof.

2. The prophylactic/therapeutic agent of claim 1, wherein the stress-induced bowel disease is stress-induced diarrhea.

3. The prophylactic/therapeutic agent of claim 1, wherein the stress-induced bowel disease is at least one kind selected from the group consisting of ulcerative colitis, Crohn',s disease, irritable bowel syndrome, functional diarrhea and stress-induced exacerbation of these bowel diseases.

4. The prophylactic/therapeutic agent of claim 1, wherein the stress-induced bowel disease is a bowel disease resulting from the release of a chemical transmitter during stress induction.

5. The prophylactic/therapeutic agent of claim 4, wherein the bowel disease resulting from the release of a chemical transmitter during stress induction is stress-induced diarrhea.

6. The prophylactic/therapeutic agent of claim 4 or 5, wherein the chemical transmitter is at least one kind selected from among catecholamine, serotonin and histamine.

7. A suppressant of the release of a chemical transmitter during stress induction, comprising, as an active ingredient, at least one kind selected from the group consisting of histidine, a peptide comprising histidine, a precursor thereof and a pharmacologically acceptable salt thereof.

8. The release suppressant of claim 7, wherein the chemical transmitter is at least one kind selected from among catecholamine, serotonin and histamine.

9. A food/beverage for prevention/amelioration of a stress-induced bowel disease, comprising, as an active ingredient, at least one kind selected from the group consisting of histidine, a peptide comprising histidine, a precursor thereof and a pharmacologically acceptable salt thereof.

10. A food/beverage comprising at least one kind selected from the group consisting of histidine, a peptide comprising histidine, a precursor thereof and a pharmacologically acceptable salt thereof, having prophylactic/ameliorating action on a stress-induced bowel disease, and bearing a label stating that it is intended to prevent/ameliorate a stress-induced bowel disease.

11. The food/beverage of claim 9 or 10, wherein the stress-induced bowel disease is stress-induced diarrhea.

12. The food/beverage of claim 9 or 10, wherein the stress-induced bowel disease is at least one kind selected from the group consisting of ulcerative colitis, Crohn's disease, irritable bowel syndrome, functional diarrhea and stress-induced exacerbation of these bowel diseases.

13. The food/beverage of claim 9 or 10, wherein the stress-induced bowel disease is a bowel disease resulting from the release of a chemical transmitter during stress induction.

14. The food/beverage of claim 13, wherein the bowel disease resulting from the release of a chemical transmitter during stress induction is stress-induced diarrhea.

15. The food/beverage of claim 13 or 14, wherein the chemical transmitter is at least one kind selected from among catecholamine, serotonin and histamine.

16. The food/beverage of any of claims 9 to 15, which is a food with health claims or a dietary supplement.

17. The food/beverage of claim 16, wherein the above-mentioned food with health claims is a food for specified health uses or a food with nutrient function claims.

18. A food/beverage comprising at least one kind selected from the group consisting of histidine, a peptide comprising histidine, a precursor thereof and a pharmacologically acceptable salt thereof, having suppressive action on the release of a chemical transmitter during stress induction, and bearing a label stating that it is intended to suppress the release of a chemical transmitter during stress induction.

19. A commercial package comprising a composition comprising, as an active ingredient, at least one kind selected from the group consisting of histidine, a peptide comprising histidine, a precursor thereof and a pharmacologically acceptable salt thereof, and a printed matter stating that the composition can be used, or should be used, to prevent/treat a stress-induced bowel disease.

20. A commercial package comprising a composition comprising, as an active ingredient, at least one kind selected from the group consisting of histidine, a peptide comprising histidine, a precursor thereof and a pharmacologically acceptable salt thereof, and a printed matter stating that the composition can be used, or should be used, to suppress the release of a chemical transmitter during stress induction.

21. A commercial package comprising a food/beverage comprising at least one kind selected from the group consisting of histidine, a peptide comprising histidine, a precursor thereof and a pharmacologically acceptable salt thereof, and a printed matter showing matters concerning its use for prevention/amelioration of a stress-induced bowel disease.

22. A commercial package comprising a food/beverage comprising at least one kind selected from the group consisting of histidine, a peptide comprising histidine, a precursor thereof and a pharmacologically acceptable salt thereof, and a printed matter showing matters concerning its use for suppression of the release of a chemical transmitter during stress induction.

23. A prophylactic or therapeutic method for a stress-induced bowel disease, comprising administering an effective amount of at least one kind selected from the group consisting of histidine, a peptide comprising histidine, a precursor thereof and a pharmacologically acceptable salt thereof to a subject of administration.

24. A method of suppressing the release of a chemical transmitter during stress induction, comprising administering an effective amount of at least one kind selected from the group consisting of histidine, a peptide comprising histidine, a precursor thereof and a pharmacologically acceptable salt thereof to a subject of administration.

25. A prophylactic or ameliorating method for a stress-induced bowel disease, comprising taking a food/beverage containing an effective amount of at least one kind selected from the group consisting of histidine, a peptide comprising histidine, a precursor thereof and a pharmacologically acceptable salt thereof to a subject of administration.

26. A method of suppressing the release of a chemical transmitter during stress induction, comprising taking a food/beverage comprising an effective amount of at least one kind selected from the group consisting of histidine, a peptide comprising histidine, a precursor thereof and a pharmacologically acceptable salt thereof to a subject of administration.

27. A use of histidine, a peptide comprising histidine, a precursor thereof or a pharmacologically acceptable salt thereof for producing a prophylactic/therapeutic agent for a stress-induced bowel disease.

28. A use of histidine, a peptide comprising histidine, a precursor thereof or a pharmacologically acceptable salt thereof for producing a suppressant of the release of a chemical transmitter during stress induction.

29. A use of histidine, a peptide comprising histidine, a precursor thereof or a pharmacologically acceptable salt thereof for producing a food/beverage having prophylactic/ameliorating action on a stress-induced bowel disease, and bearing a label stating that it is intended to prevent/ameliorate a stress-induced bowel disease.

30. A use of histidine, a peptide comprising histidine, a precursor thereof or a pharmacologically acceptable salt thereof for producing a food/beverage having suppressive action on the release of a chemical transmitter during stress induction, and bearing a label stating that it is intended to suppress the release of a chemical transmitter during stress induction.
